# EUROPÄISCHE PATENTSCHRIFT

(11) **EP 3 077 057 B1**
(45) Veröffentlichungstag und Bekanntmachung des Hinweises auf die Patenterteilung: **07.11.2018**
(21) Anmeldenummer: 14777255.2
(22) Anmeldetag: 01.09.2014
(51) Int. Cl.: A61Q 5/06, A61K 8/85

(54) **LÄNGER HALTENDES HAARSPRAY**
LONGER LASTING HAIRSPRAY
FIXATIVE DES CHEVEUX À LONG DURANCE

(30) Priorität: 04.12.2013 DE 102013224869
(43) Veröffentlichungstag der Anmeldung: 12.10.2016
(73) Patentinhaber: Henkel AG & Co. KGaA, 40589 Düsseldorf (DE)
(72) Erfinder: KNAPPE, Thorsten, 22869 Schenefeld (DE); SCHMARJE, Susanne, 22307 Hamburg (DE)
(86) Internationale Anmeldenummer: PCT/DE2014/200435
(87) Internationale Veröffentlichungsnummer: WO 2015/081935

(56) Entgegenhaltungen:
- WO-A2-2010/122286
- WO-A2-2010/130626
- TIMOTHY GAO ET.AL.: "Study of hair shine and hair surface smoothness", JOURNAL OF COSMETIC SCIENCE MARCH/APRIL 2009, Bd. 60, 2009, Seiten 187-197, XP002733643,

## Beschreibung

Die vorliegende Erfindung betrifft eine anwendungsfertige konfektionierte Zubereitung zur Behandlung keratinischer Fasern, insbesondere menschlicher Haare in Form eines Sprays, sowie die Verwendung dieser Zubereitung zur Behandlung keratinischer Fasern, insbesondere menschlicher Haare.

Haarsprays enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden.

Das bzw. die Polymer(e) dienen dazu, der Frisur einen möglichst lang anhaltenden Halt zu geben. Diese Frisurstabilität geht oft mit einem Verlust an Haargefühl einher, d.h. die Haare verlieren ihre Weichheit und ihren angenehmen Griff, wobei es im Extremfall zum Gefühl einer "helmartigen" Frisur kommt.

Neben einem hohen Haltegrad müssen Stylingmittel jedoch eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Die WO2010/130626 A2und die WO2010/122286 A2 offenbaren in Beispiel 5.7 bzw. auf Seite 6, Zeile 17ff Haarsprayzusammensetzungen, welche PPG-3 Benzyl Ester Myristate, Treibmittel und Polymere enthalten.

Die Verbesserung des Glanzes von Haaren durch PPG-Benzyl-Fettsäureester wird in "Study of hair shine and hair surface smoothness", Journal of Cosmetic Science, March/April 2009, Bd. 60, p. 187-197 offenbart.

Der vorliegenden Erfindung lag die Aufgabe zugrunde, Haarsprays bereitzustellen, welche dem Haar einen angenehmen Griff und ein gepflegtes Haargefühl verleihen, ohne dass der lang anhaltende Halt negativ beeinflusst wird. Im Idealfall soll sogar eine Verbesserung des Haltes erzielt werden.

Es wurde nun gefunden, dass sich das Haargefühl, die Leichtigkeit der Frisur sowie der Griff der Haare auch unter weiterer Verbesserung des Haltes verbessern lassen, wenn bestimmte Ester in die Haarsprays inkorporiert werden.

Ein erster Gegenstand der Erfindung ist daher ein Haarspray, umfassend einen Druckbehälter und - darin befindlich - eine Aerosolzusammensetzung, die - bezogen auf ihr Gewicht -
a) 20 bis 80 Gew.-% mindestens eines Treibmittels;
b) 0,1 bis 20 Gew.-% mindestens eines festigenden Polymers;
c) 0,01 bis 5 Gew.-% Ester der Formel (I) 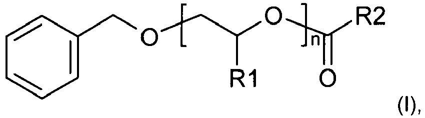 in der
   - R1: für-H oder-CH₃,
   - R2: für einen geradkettigen oder verzweigten Alkylrest mit 7 bis 15 Kohlenstoffatomen,
   - n: für eine ganze Zahl aus der Gruppe 1, 2, 3, 4, 5,6 ,7, 8
   steht, enthält
   mit der Maßgabe, dass
   die Aerosolzusammensetzung - bezogen auf ihr Gewicht - 0,1 bis 10 Gew Copolymere(e) aus N-Octylacrylamid/Acrylsäure/tert.-Butylaminoethylmethacrylat (INCI-Bezeichnung: Octylacrylamide / Acrylates / Butylaminoethyl Methacrylate Copolymer) enthält.

Als ersten wesentlichen Bestandteil enthalten die erfindungsgemäßen Haarsprayzusammensetzungen 20 bis 80 Gew.-% mindestens eines Treibmittels.

Erfindungsgemäß geeignete Treibmittel (Treibgase) sind beispielsweise Propan, n-Butan, iso-Butan, Dimethylether (DME), Stickstoff, Luft, Lachgas, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Dimethylether, Propan, n-Butan, iso-Butan sowie Mischungen dieser Treibgase. Ganz besonders bevorzugt ist der Einsatz von Propan/Butan Gemischen oder Isobutan.

Besonders bevorzugte erfindungsgemäße Haarsprays sind dadurch gekennzeichnet, dass die Aerosolzusammensetzung - bezogen auf ihr Gewicht - 20 bis 80 Gew.-%, vorzugsweise 22,5 bis 77,5 Gew.-%, weiter bevorzugt 25 bis 75 Gew.-%, noch weiter bevorzugt 27,5 bis 72,5 Gew.-% und insbesondere 30 bis 70 Gew.-% Treibmittel oder Treibmittelgemisch, ausgewählt aus Dimethylether, HFO1234yf, HFO1234ze, Propan, Propen, n-Butan, iso-Butan, iso-Buten, n-Pentan, Penten, iso-Pentan und iso-Penten oder deren Mischungen, enthält.

Vorteilhafterweise wird das Treibmittel so ausgewählt, dass es gleichzeitig als Lösungsmittel für weitere Inhaltsstoffe wie beispielsweise Öl- und Wachskomponenten, den Fettstoffen (D) dienen kann. Das Treibmittel kann dann als Lösungsmittel für diese letztgenannten Komponenten dienen, wenn diese bei 20 °C zu mindestens 0,5 Gew.-%, bezogen auf das Treibmittel, in diesem löslich sind.

Gemäß einer bevorzugten Ausführungsform enthalten die erfindungsgemäßen Zubereitungen die genannten Kohlenwasserstoffe, Dimethylether oder Mischungen der genannten Kohlenwasserstoffe mit Dimethylether als einziges Treibmittel. Die Erfindung umfasst aber ausdrücklich auch die Mitverwendung von Treibmittel vom Typ der Fluorchlorkohlenwasserstoffe, insbesondere aber der Fluorkohlenwasserstoffe.

Ganz besonders bevorzugte erfindungsgemäße Kombinationen sind dadurch gekennzeichnet, daß das treibmittelhaltige Haarbehandlungsmittel bezogen auf sein Gewicht 7,5 bis 57,5 Gew.-%, vorzugsweise 10 bis 55 Gew.-%, weiter bevorzugt 15 bis 52,5 Gew.-%, noch weiter bevorzugt 17,5 bis 55 Gew.-%, besonders bevorzugt 20 bis 50 Gew.-% und insbesondere 25 bis 45 Gew.-% mindestens eines Treibmittels, ausgewählt aus n-Propan, n-Butan, iso-Butan, n-Pentan, Dimethylether und deren Mischungen, enthält.

Als zweiten wesentlichen Bestandteil enthalten die erfindungsgemäßen Haarsprayzusammensetzungen 0,1 bis 20 Gew.-% mindestens eines festigenden Polymers
Dabei können diese filmbildenden und/oder festigenden Polymere sowohl permanent als auch temporär kationisch, anionisch, nichtionisch oder amphoter sein. Weiterhin umfasst die vorliegende Erfindung auch die Erkenntnis, dass bei der Verwendung von mindestens zwei filmbildenden und/oder festigenden Polymeren diese selbstverständlich unterschiedliche Ladungen aufweisen können. Erfindungsgemäß bevorzugt kann es sein, wenn ein ionisches filmbildendes und/oder festigendes Polymer mit einem amphoteren und/oder nichtionischem filmbildenden und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft einige filmbildende Polymere beschrieben werden. Es wird an dieser Stelle jedoch explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Zu den bevorzugten Eigenschaften der filmbildenden Polymeren zählt die Filmbildung. Unter filmbildenden Polymeren sind solche Polymere zu verstehen, welche beim Trocknen einen kontinuierlichen Film auf der Haut, dem Haar oder den Nägeln hinterlassen. Derartige Filmbildner können in den unterschiedlichsten kosmetischen Produkten wie beispielsweise Gesichtsmasken, Make-up, Haarfestigern, Haarsprays, Haargelen, Haarwachsen, Haarkuren, Shampoos oder Nagellacken verwendet werden. Bevorzugt sind insbesondere solche Polymere, die eine ausreichende Löslichkeit in Alkohol oder Wasser/Alkohol-Gemischen besitzen, um in dem erfindungsgemäßen Mittel in vollständig gelöster Form vorzuliegen. Die filmbildenden Polymere können synthetischen oder natürlichen Ursprungs sein.

Unter filmbildenden Polymeren werden weiterhin erfindungsgemäß solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden. Die filmbildenden Polymere können dabei sowohl anionisch, amphoter, nicht-ionisch, permanent kationisch oder temporär kationisch geladen sein.

Geeignete synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C₁- bis C₇-Alkylgruppen, besonders bevorzugt C₁- bis C₃-Alkylgruppen sind.

Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/Polypropylenglykol-Copolymere, die beispielsweise, unter den Handelsbezeichnungen Ucon® der Union Carbide vertrieben werden.

Geeignete natürliche filmbildende Polymere sind z.B. Cellulosederivate, z. B. Hydroxypropylcellulose mit einem Molekulargewicht von 30.000 bis 50.000 g/mol, welche beispielsweise unter der Handelsbezeichnung Nisso SI® von der Firma Lehmann & Voss, Hamburg, vertrieben wird.

Festigende Polymere tragen zum Halt und/oder zum Aufbau des Haarvolumens und der Haarfülle der Gesamtfrisur bei. Diese sogenannten festigenden Polymere sind gleichzeitig auch filmbildende Polymere und daher generell typische Substanzen für formgebende Haarbehandlungsmittel wie Haarfestiger, Haarschäume, Haarwachse, Haarsprays. Die Filmbildung kann dabei durchaus punktuell sein und nur einige Fasern miteinander verbinden.

Substanzen, welche dem Haar weiterhin hydrophobe Eigenschaften verleihen, sind hierbei bevorzugt, weil sie die Tendenz des Haares, Feuchtigkeit, also Wasser, zu absorbieren, verringern. Dadurch wird das schlaffe Herunterhängen der Haarsträhnen vermindert und somit ein langanhaltender Frisurenaufbau und -erhalt gewährleistet. Als Testmethode hierfür wird häufig der sogenannte curlretention - Test angewendet. Diese polymeren Substanzen können weiterhin erfolgreich in leave-on und rinse-off Haarkuren oder Shampoos eingearbeitet werden. Da Polymere häufig multifunktional sind, das heißt mehrere anwendungstechnisch erwünschte Wirkungen zeigen, finden sich zahlreiche Polymere in mehreren auf die Wirkungsweise eingeteilten Gruppen, so auch im CTFA Handbuch. Wegen der Bedeutung gerade der festigenden Polymere sollen diese daher explizit in Form ihrer INCI - Namen aufgelistet werden. In dieser Liste der erfindungsgemäß bevorzugt zu verwendenden Polymere finden sich somit selbstverständlich gerade auch die genannten filmbildenden Polymere wieder.

Beispiele für gebräuchliche filmbildende, festigende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate.

Eine besonders ausgeprägte Verbesserung des Frisurenhalts lässt sich erzielen, wenn ein filmbildendes und/oder festigendes Polymer eingesetzt wird, das spröde, harte Polymerfilme ausbildet. Vorzugsweise werden daher entsprechende filmbildende und/oder festigende Polymere verwendet. Vorzugsweise enthält das erfindungsgemäße Mittel daher mindestens ein filmbildendes und/oder festigendes Polymer ausgewählt aus
- Aminomethylpropanolsalzen von Copolymeren des Allylmethacrylats mit einem oder mehreren Monomeren ausgewählt aus Acrylsäure, Methacrylsäure, Acrylsäureester und Methacrylsäureester,
- Vinylpyrrolidon-Vinylacetat-Copolymeren,
- Vinylpyrrolidon-Vinylcaprolactam-Dimethylaminopropylacrylamid-Copolymeren,
- Copolymeren des Octylacrylamids mit t-Butylaminoethylmethacrylat und zwei oder mehr Monomeren ausgewählt aus Acrylsäure, Methacrylsäure, Acrylsäureester und Methacrylsäureester, und
- Copolymeren der C₁₋₂-Alkylsuccinate mit Hydroxyalkylacrylaten und einem oder mehreren Monomeren ausgewählt aus Acrylsäure, Methacrylsäure, Acrylsäureester und Methacrylsäureester.

Entsprechende filmbildende und/oder festigende Polymere sind kommerziell erhältlich.

Besonders bevorzugt enthält das erfindungsgemäße Mittel als filmbildendes und/oder festigendes Polymer ein Aminomethylpropanolsalz eines Copolymers des Allylmethacrylats mit einem oder mehreren Monomeren, ausgewählt aus Acrylsäure, Methacrylsäure, Acrylsäureester und Methacrylsäureester.

Vorzugsweise handelt es sich bei den genannten Acrylsäureestern und Methacrylsäureestern um C₁-C₁₂-Alkylacrylate und C₁-C₁₂-Alkylmethacrylate, besonders bevorzugt um Methylacrylat, Ethylacrylat, Propylacrylat, Methylmethacrylat, Ethylmethacrylat, Propylmethacrylat und deren Mischungen.

Als Aminomethylpropanolsalz von Copolymeren des Allylmethacrylats mit einem oder mehreren Monomeren ausgewählt aus Acrylsäure, Methacrylsäure, Acrylsäureester und Methacrylsäureester, wird vorzugsweise das Copolymer mit der INCI-Bezeichnung AMP-Acrylates/Allyl Methacrylate Copolymer eingesetzt, das von der Firma Noveon unter der Bezeichnung Fixate™ G-100 vertrieben wird. Das erfindungsgemäße Mittel enthält dieses Copolymer ganz besonders bevorzugt.

Ein bevorzugtes Vinylpyrrolidon-Vinylacetat-Copolymer ist das PVP/VA Copolymer 60-40 W (INCI-Bezeichnung: VP/VA Copolymer, Aqua, Laurtrimonium Chloride).

Als Vinylpyrrolidon-Vinylcaprolactam/Dimethylaminopropylacrylamid-Copolymer wird vorzugsweise das von ISP unter der Bezeichnung Aquaflex SF 40 erhältliche Copolymer mit der INCI-Bezeichnung VP/Vinyl Caprolactam/DMAPA Acrylates Copolymer eingesetzt.

Ein bevorzugtes Copolymer des Octylacrylamids mit t-Butylaminoethylmethacrylat und zwei oder mehr Monomeren ausgewählt aus Acrylsäure, Methacrylsäure, Acrylsäureester und Methacrylsäureester, ist das von National Starch unter der Bezeichnung Amphomer® erhältliche Copolymer mit der INCI-Bezeichnung Octylacrylamide/Acrylates Butylaminoethyl Methacrylates Copolymer.

Als Copolymer der C₁₋₂-Alkylsuccinate mit Hydroxyalkylacrylaten und einem oder mehreren Monomeren ausgewählt aus Acrylsäure, Methacrylsäure, Acrylsäureester und Methacrylsäureester, ist das von ISP unter der Bezeichnung Allianz™ LT 120 erhältliche Copolymer mit der INCI-Bezeichnung Acrylates/C1-2 Succinates/Hydroxyacrylates Copolymer bevorzugt. Erfindungsgemäß besonders bevorzugte Haarsprays sind dadurch gekennzeichnet, daß die Aerosolzusammensetzung - bezogen auf ihr Gewicht - festigende(s) Polymer(e) in einer Gesamtmenge von 0,2 Gew.-% bis 17,5 Gew.-%, vorzugsweise von 0,5 Gew.-% bis 15 Gew.-%, besonders bevorzugt von 2,0 Gew.-% bis 10,0 Gew.-% und insbesondere von 3,0 bis 5,0 Gew.-%, jeweils bezogen auf das Gewicht des Mittels, enthält. Ganz besonders bevorzugte erfindungsgemäße Haarsprays sind, dadurch gekennzeichnet, daß die Aerosolzusammensetzung mindestens ein festigendes Polymer ausgewählt aus
- nichtionischen Polymeren auf Basis ethylenisch ungesättigter Monomere, insbesondere aus
- Homopolymeren des N-Vinylpyrrolidons,
- nichtionischen Copolymeren des N-Vinylpyrrolidons,
- Homopolymeren und nichtionischen Copolymeren des N-Vinylcaprolactams,
- Copolymeren des (Meth)acrylamids,
- Polyvinylalkohol, Polyvinylacetat,
- Chitosan und Derivaten des Chitosans,
- kationischen Cellulosederivaten,
- kationischen Copolymeren des 3-(C₁ bis C₆)-Alkyl-1-vinyl-imidazoliniums,
- Homopolymeren und Copolymeren enthaltend die Struktureinheit der Formel (M-1) 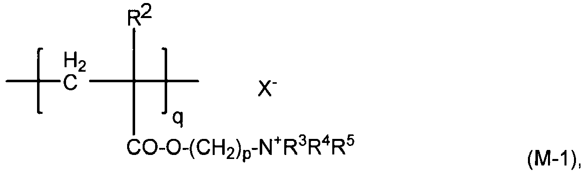 in der R²= -H oder -CH₃ ist, R³, R⁴ und R⁵ unabhängig voneinander ausgewählt sind aus (C₁ bis C₄)-Alkyl-, (C₁ bis C₄)-Alkenyl-oder (C₂ bis C₄)-Hydroxyalkylgruppen, p = 1, 2, 3 oder 4, q eine natürliche Zahl und X⁻ ein physiologisch verträgliches organisches oder anorganisches Anion ist,
- anionischen Polymeren, welche Carboxylat- und/oder Sulfonatgruppen aufweisen,
- anionischen Polyurethanen
enthält.

Es hat sich gezeigt, dass ein bestimmtes Copolymer in der erfindungsgemäßen Kombination außerordentlich gute Haltegrade liefert, die durch den Einsatz der nachfolgend beschriebenen Ester nicht nur verstärkt, sondern durch ein exzellentes Haargefühl ergänzt werden. Erfindungsgemäß bevorzugte Haarsprays sind daher dadurch gekennzeichnet, dass die Aerosolzusammensetzung - bezogen auf ihr Gewicht - 0,25 bis 9 Gew.-%, weiter bevorzugt 0,5 bis 8 Gew.-%, besonders bevorzugt 0,75 bis 7 Gew.-% und insbesondere 1 bis 6 Gew.-% Copolymere(e) aus N-Octylacrylamid/Acrylsäure/tert.-Butylaminoethylmethacrylat (INCI-Bezeichnung: Octylacrylamide / Acrylates / Butylaminoethyl Methacrylate Copolymer) enthält.

Als dritten wesentlichen Bestandteil enthalten die erfindungsgemäßen Haarsprayzusammensetzungen 0,01 bis 5 Gew.-% Ester der Formel (I) 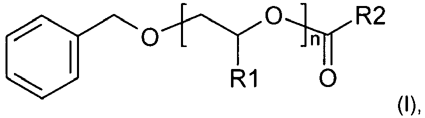 in der
- R1: für -H oder-CH₃,
- R2: für einen geradkettigen oder verzweigten Alkylrest mit 7 bis 15 Kohlenstoffatomen,
- n: für eine ganze Zahl aus der Gruppe 1, 2, 3, 4, 5,6 ,7, 8
steht.

Vorzugsweise wird der bzw. werden die Ester der Formel (I) innerhalb engerer Mengenbereiche eingesetzt. Bevorzugte Haarsprays sind dadurch gekennzeichnet, daß die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (I) enthält.

In Formel (I) steht R1 vorzugsweise für eine Methylgruppe, so daß bevorzugte Haarsprays dadurch gekennzeichnet sind, daß die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (la) enthält 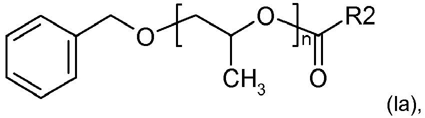 in der
- R2: für einen geradkettigen oder verzweigten Alkylrest mit 7 bis 15 Kohlenstoffatomen,
- n: für eine ganze Zahl aus der Gruppe 1, 2, 3, 4, 5, 6 ,7, 8
steht.

In Formel (Ia) steht n bevorzugt für eine ganze Zahl aus der Gruppe 1, 2, 3, 4, 5, 6, d.h. bevorzugte Ester lassen sich durch die Formeln (Ia-1) bis (Ia-6) beschreiben: 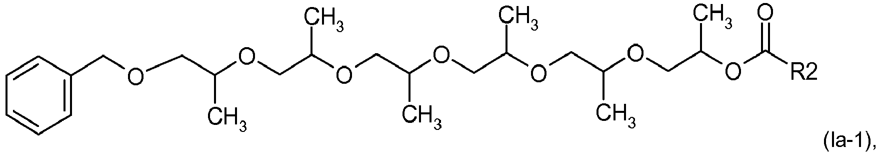 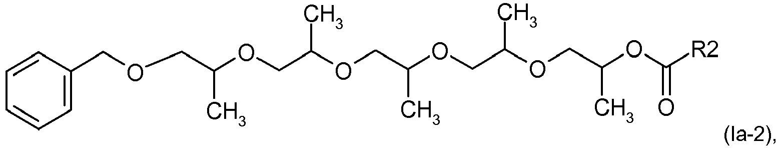 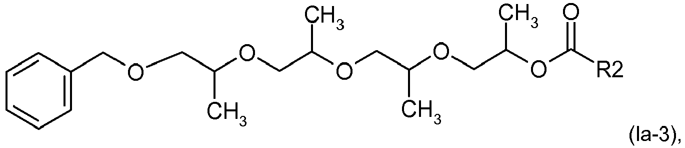 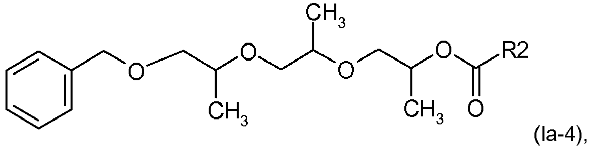 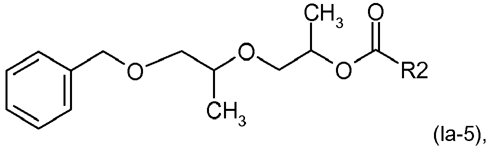 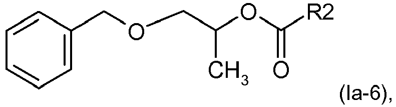 in denen R2 jeweils für einen geradkettigen oder verzweigten Alkylrest mit 7 bis 15 Kohlenstoffatomen steht.

Ganz besonders bevorzugte Reste R2 stehen für Tridecyl- oder Ethylhexylreste, so daß besonders bevorzugte Haarsprays dadurch gekennzeichnet sind, daß die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (Ib) enthält 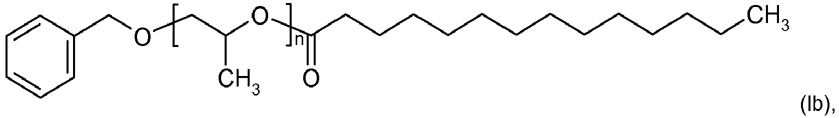 in der n für eine ganze Zahl aus der Gruppe 1, 2, 3 oder 4, vorzugsweise für 2 oder 3 und insbesondere für 3, steht und weitere ebenfalls besonders bevorzugte Haarsprays dadurch gekennzeichnet sind, daß die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (Ic) enthält 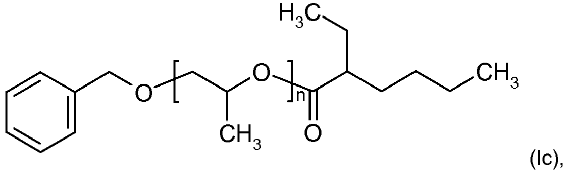 in der n für eine ganze Zahl aus der Gruppe 1, 2, 3 oder 4, vorzugsweise für 2 oder 3 und insbesondere für 3, steht. Ganz besonders bevorzugt einzusetzende Ester sind demnach die Verbindungen (Ib-1) und (Ic-1):

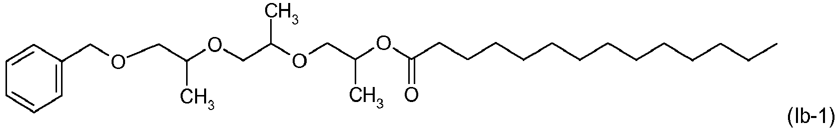

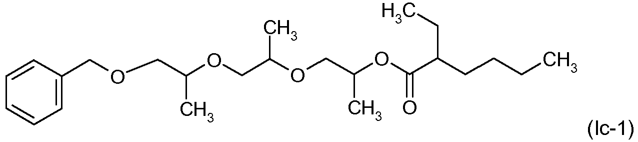

Neben den zuvor beschriebenen Copolymeren und Estern können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe.

Als bevorzugten Bestandteil enthalten die erfindungsgemäßen kosmetischen Mittel mindestens eine quartäre Ammoniumverbindung. Als quartäre Ammoniumverbindung können monomere oder polymere Wirkstoffe eingesetzt werden.

Aus der Vielzahl an möglichen monomeren quartären Ammoniumverbindungen haben sich die Verbindungen aus den Gruppen:
- Trimethylalkylammoniumhalogenide;
- der Esterquats
- der quartären Imidazoline
als besonders wirkungsvoll erwiesen.

Zur Gruppe der Trimethylalkylammoniumhalogenide zählen insbesondere die Verbindungen der Formel (Tkat1-1).

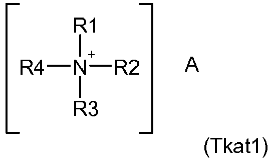

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat. Bevorzugte kosmetische Mittel enthalten eine monomere quartäre Ammoniumverbindung aus der Gruppe der Trimethylalkylammoniumhalogenide.

Weitere erfindungsgemäß besonders bevorzugte quartären Ammoniumverbindungen sind die kationischen Betainestern der Formel (Tkat1-2.1).

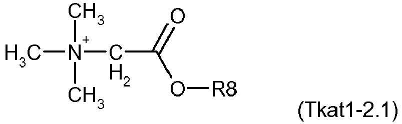

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe sind quartäre Imidazolinverbindungen. Die im Folgenden dargestellte Formel (Tkat2) zeigt die Struktur dieser Verbindungen.

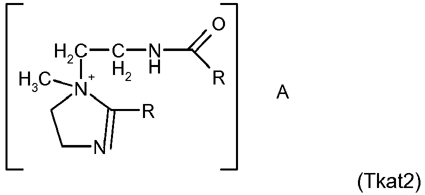

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCI - Bezeichnungen Quaternium-27, Quaternium-72, Quaternium-83, Quaternium-87 und Quaternium-91 erhältlich. Höchst bevorzugt ist erfindungsgemäß Quaternium-91.

In Bezug auf die kosmetische Wirkung haben sich kosmetische Mittel als vorteilhaft erwiesen, bei denen der Gewichtsanteil der monomeren quartären Ammoniumverbindung am Gesamtgewicht des Mittels 0,05 bis 3,0 Gew.-%, vorzugsweise 0,1 bis 2,0 Gew.-% und insbesondere 0,2 bis 1,0 Gew.-% beträgt.

Als geeignete Hilfs- und Zusatzstoffe sind insbesondere zusätzliche Pflegestoffe zu nennen.

Als Pflegestoff einer anderen Verbindungsklasse kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von C₆ - C₃₀ - Fettsäuren mit C₂ - C₃₀ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/- caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind. In Bezug auf die kosmetische Wirkung haben sich kosmetische Mittel als vorteilhaft erwiesen, bei denen der Gewichtsanteil des Ölkörpers am Gesamtgewicht des Mittels 0,01 bis 5,0 Gew.-%, vorzugsweise 0,02 bis 4,0 Gew.-% und insbesondere 0,05 bis 2,0 Gew.-% beträgt.

Die erfindungsgemäße Kombination umfaßt weiterhin einen Spender (Druckbehälter), aus welchem das in ihm enthaltene treibgashaltige Haarbehandlungsmittel mit Hilfe des Treibmittels ausgesprüht wird. Erfindungsgemäß bevorzugt umfaßt der Spender ein Ventil, welches eine Ventilöffnung beinhaltet, die als Kegelbohrung ausgestaltet ist und deren Durchmesser maximal 0,4 mm beträgt. Zusätzlich beinhaltet das Ventil vorzugsweise eine Drosselung, welche einen Innendurchmesser von maximal 0,3 mm aufweist, aber besitzt keine Seitenbohrung ("Gasphasenbohrung"). Vorzugsweise beträgt der Durchmesser der Kegelbohrung 0,15 bis 0,4 mm, vorzugsweise 0,175 bis 0,375 mm, weiter bevorzugt 0,2 bis 0,35 mm und insbesondere 0,25 bis 0,3 mm. Vorzugsweise beträgt auch die Tiefe der Kegelbohrung maximal 0,3 mm. Bevorzugte erfindungsgemäße Kombinationen sind weiter dadurch gekennzeichnet, daß die Tiefe der Kegelbohrung 0,1 bis 0,3 mm, vorzugsweise 0,125 bis 0,275 mm, weiter bevorzugt 0,15 bis 0,25 mm und insbesondere 0,2 bis 0,25 mm beträgt.

Die Ausgestaltung des Ventils umfaßt weiterhin vorzugsweise eine Drosselung, die den Durchsatz begrenzt. Die Drosselung befindet sich dabei entweder im Stem des Ventiles oder im Sprühkopf. Diese Drosselung weist in bevorzugten Ausführungsformen der vorliegenden Erfindung einen Innendurchmesser von 0,1 bis 0,3 mm, vorzugsweise 0,125 bis 0,275 mm, weiter bevorzugt 0,15 bis 0,25 mm und insbesondere 0,2 bis 0,25 mm auf. Die erfindungsgemäßen Zusammensetzungen können in handelsüblichen Aerosoldosen verpackt sein. Die Dosen können aus Weißblech oder aus Aluminium sein. Weiterhin können die Dosen innen beschichtet sein, um die Gefahr der Korrosion so gering wie möglich zu halten. Auch der Einsatz von Innenbeuteln in Dosen ist problemlos möglich. Die Dosen sind mit einem geeigneten Sprühkopf ausgestattet. Je nach Sprühkopf sind Ausstoßraten, bezogen auf voll gefüllte Dosen, von 0,1 g/s bis 5,0 g/s möglich.

Ein weiterer Gegenstand der vorliegenden Erfindung ist ein Verfahren zur Frisurengestaltung, bei dem eine Aerosolzusammensetzung, welche Ester der Formel (I) 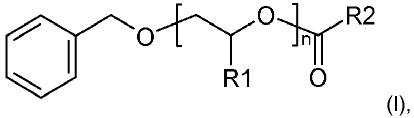 in der
- R1: für -H oder -CH₃,
- R2: für einen geradkettigen oder verzweigten Alkylrest mit 7 bis 15 Kohlenstoffatomen,
- n: für eine ganze Zahl aus der Gruppe 1, 2, 3, 4, 5,6 ,7, 8
steht, enthält, mittels eines Treibmittels oder Treibmittelgemisches aus einem Druckbehälter auf keratinische Fasern aufgebracht wird.

Bevorzugte erfindungsgemäße Verfahren setzen die Ester dabei in Kombination mit mindestens einem festigenden Polymer ein. Auch bezüglich weiterer bevorzugter Ausführungsformen des erfindungsgemäßen Verfahrens gilt mutatis mutandis das zu den erfindungsgemäßen Mitteln Gesagte.

## Patentansprüche

1. Haarspray, umfassend einen Druckbehälter und - darin befindlich - eine Aerosolzusammensetzung, die - bezogen auf ihr Gewicht -
a) 20 bis 80 Gew.-% mindestens eines Treibmittels;
b) 0,1 bis 20 Gew.-% mindestens eines festigenden Polymers;
c) 0,01 bis 5 Gew.-% Ester der Formel (I) 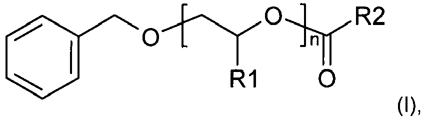 in der
R1 für -H oder-CH₃,
R2 für einen geradkettigen oder verzweigten Alkylrest mit 7 bis 15 Kohlenstoffatomen,
n für eine ganze Zahl aus der Gruppe 1, 2, 3, 4, 5,6 ,7, 8
steht, enthält
mit der Maßgabe daß
die Aerosolzusammensetzung - bezogen auf ihr Gewicht - 0,1 bis 10 Gew Copolymere(e) aus N-Octylacrylamid/Acrylsäure/tert.-Butylaminoethylmethacrylat (INCI-Bezeichnung: Octylacrylamide / Acrylates / Butylaminoethyl Methacrylate Copolymer) enthält.

2. Haarspray nach Anspruch 1, **dadurch gekennzeichnet, dass** die Aerosolzusammensetzung - bezogen auf ihr Gewicht - 20 bis 80 Gew.-%, vorzugsweise 22,5 bis 77,5 Gew.-%, weiter bevorzugt 25 bis 75 Gew.-%, noch weiter bevorzugt 27,5 bis 72,5 Gew.-% und insbesondere 30 bis 70 Gew.-% Treibmittel oder Treibmittelgemisch, ausgewählt aus Dimethylether, HFO1234yf, HFO1234ze, Propan, Propen, n-Butan, iso-Butan, iso-Buten, n-Pentan, Penten, iso-Pentan und iso-Penten oder deren Mischungen, enthält.

3. Haarspray nach einem der Ansprüche 1 oder 2, **dadurch gekennzeichnet, dass** es Propan, Propen, n-Butan, iso-Butan, iso-Buten, n-Pentan, Penten, iso-Pentan und iso-Penten, Dimethylether oder Mischungen der genannten Kohlenwasserstoffe mit Dimethylether als einziges Treibmittel enthält.

4. Haarspray nach einem der Ansprüche 1 bis 3, **dadurch gekennzeichnet, dass** die Aerosolzusammensetzung - bezogen auf ihr Gewicht - festigende(s) Polymer(e) in einer Gesamtmenge von 0,2 Gew.-% bis 17,5 Gew.-%, vorzugsweise von 0,5 Gew.-% bis 15 Gew.-%, besonders bevorzugt von 2,0 Gew.-% bis 10,0 Gew.-% und insbesondere von 3,0 bis 5,0 Gew.-%, jeweils bezogen auf das Gewicht des Mittels, enthält.

5. Haarspray nach einem der Ansprüche 1 bis 4, **dadurch gekennzeichnet, dass** die Aerosolzusammensetzung mindestens ein festigendes Polymer ausgewählt aus
- nichtionischen Polymeren auf Basis ethylenisch ungesättigter Monomere, insbesondere aus
- Homopolymeren des N-Vinylpyrrolidons,
- nichtionischen Copolymeren des N-Vinylpyrrolidons,
- Homopolymeren und nichtionischen Copolymeren des N-Vinylcaprolactams,
- Copolymeren des (Meth)acrylamids,
- Polyvinylalkohol, Polyvinylacetat,
- Chitosan und Derivaten des Chitosans,
- kationischen Cellulosederivaten,
- kationischen Copolymeren des 3-(C₁ bis C₆)-Alkyl-1-vinyl-imidazoliniums,
- Homopolymeren und Copolymeren enthaltend die Struktureinheit der Formel (M-1) 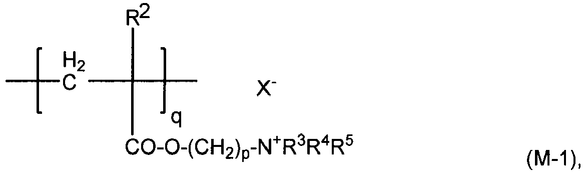 in der R²= -H oder -CH₃ ist, R³, R⁴ und R⁵ unabhängig voneinander ausgewählt sind aus (C₁ bis C₄)-Alkyl-, (C₁ bis C₄)-Alkenyl- oder (C₂ bis C₄)-Hydroxyalkylgruppen, p = 1, 2, 3 oder 4, q eine natürliche Zahl und X⁻ ein physiologisch verträgliches organisches oder anorganisches Anion ist,
- anionischen Polymeren, welche Carboxylat- und/oder Sulfonatgruppen aufweisen,
- anionischen Polyurethanen
enthält.

6. Haarspray nach einem der Ansprüche 1 bis 5, **dadurch gekennzeichnet, dass** es mindestens ein filmbildendes und/oder festigendes Polymer ausgewählt aus
- Aminomethylpropanolsalzen von Copolymeren des Allylmethacrylats mit einem oder mehreren Monomeren ausgewählt aus Acrylsäure, Methacrylsäure, Acrylsäureester und Methacrylsäureester,
- Vinylpyrrolidon-Vinylacetat-Copolymeren,
- Vinylpyrrolidon-Vinylcaprolactam-Dimethylaminopropylacrylamid-Copolymeren,
- Copolymeren des Octylacrylamids mit t-Butylaminoethylmethacrylat und zwei oder mehr Monomeren ausgewählt aus Acrylsäure, Methacrylsäure, Acrylsäureester und Methacrylsäureester, und
- Copolymeren der C₁₋₂-Alkylsuccinate mit Hydroxyalkylacrylaten und einem oder mehreren Monomeren ausgewählt aus Acrylsäure, Methacrylsäure, Acrylsäureester und Methacrylsäureester
enthält.

7. Haarspray nach einem der Ansprüche 1 bis 6, **dadurch gekennzeichnet, dass** es als filmbildendes und/oder festigendes Polymer ausgewählt aus ein Aminomethylpropanolsalz eines Copolymers des Allylmethacrylats mit einem oder mehreren Monomeren, ausgewählt aus Acrylsäure, Methacrylsäure, Acrylsäureester und Methacrylsäureester enthält.

8. Haarspray nach einem der Ansprüche 1 bis 7, **dadurch gekennzeichnet, dass** die Aerosolzusammensetzung - bezogen auf ihr Gewicht - 0,25 bis 9 Gew.-%, weiter bevorzugt 0,5 bis 8 Gew.-%, besonders bevorzugt 0,75 bis 7 Gew.-% und insbesondere 1 bis 6 Gew.-% Copolymere(e) aus N-Octylacrylamid/Acrylsäure/tert.-Butylaminoethylmethacrylat (INCI-Bezeichnung: Octylacrylamide / Acrylates / Butylaminoethyl Methacrylate Copolymer) enthält.

9. Haarspray nach einem der Ansprüche 1 bis 8, **dadurch gekennzeichnet, dass** die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (I) enthält.

10. Haarspray nach einem der Ansprüche 1 bis 9, **dadurch gekennzeichnet, dass** die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (la) enthält 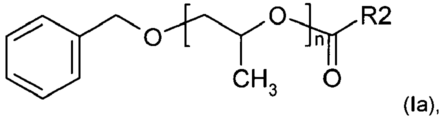 in der
R2 für einen geradkettigen oder verzweigten Alkylrest mit 7 bis 15 Kohlenstoffatomen,
n für eine ganze Zahl aus der Gruppe 1, 2, 3, 4, 5,6 ,7, 8
steht.

11. Haarspray nach einem der Ansprüche 1 bis 10, **dadurch gekennzeichnet, dass** die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (Ib) enthält 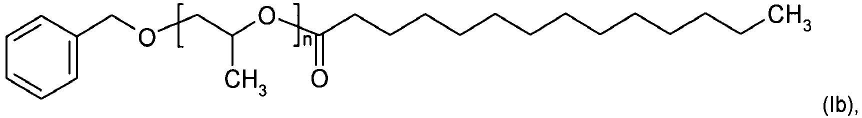 in der n für eine ganze Zahl aus der Gruppe 1, 2, 3 oder 4, vorzugsweise für 2 oder 3 und insbesondere für 3, steht.

12. Haarspray nach einem der Ansprüche 1 bis 11, **dadurch gekennzeichnet, dass** die Aerosolzusammensetzung - bezogen auf ihr Gewicht - 0,11 bis 4,5 Gew.-%, vorzugsweise 0,12 bis 4 Gew.-%, weiter bevorzugt 0,13 bis 3 Gew.-%, besonders bevorzugt 0,14 bis 2,5 Gew.-% und insbesondere 0,15 bis 2 Gew.-% Ester der Formel (Ic) enthält 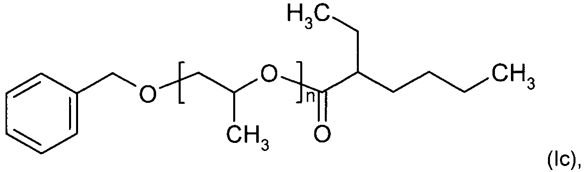 in der n für eine ganze Zahl aus der Gruppe 1, 2, 3 oder 4, vorzugsweise für 2 oder 3 und insbesondere für 3, steht.

13. Verfahren zur Frisurengestaltung, **dadurch gekennzeichnet, dass** eine Aerosolzusammensetzung, gemäss Anspruch 1 welche Ester der Formel (I) 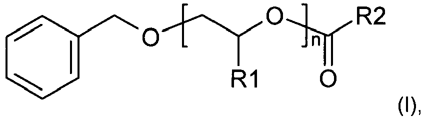 in der
R1 für -H oder-CH₃,
R2 für einen geradkettigen oder verzweigten Alkylrest mit 7 bis 15 Kohlenstoffatomen,
n für eine ganze Zahl aus der Gruppe 1, 2, 3, 4, 5,6 ,7, 8
steht, enthält, mittels eines Treibmittels oder Treibmittelgemisches aus einem Druckbehälter auf keratinische Fasern aufgebracht wird.

## Claims

1. A hairspray comprising a pressurized container and an aerosol composition located therein that contains, based on the weight thereof,
a) 20 to 80 wt.% at least one propellant;
b) 0.1 to 20 wt. % at least one setting polymer;
c) 0.01 to 5 wt.% esters of formula (I) 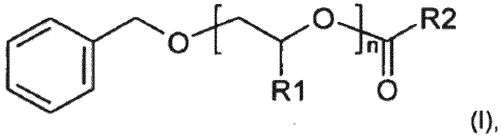 in which
R1 represents -H or-CH₃,
R2 represents a straight-chain or branched alkyl functional group having 7 to 15 carbon atoms,
n represents an integer from the group of 1, 2, 3, 4, 5, 6, 7 and 8,
with the proviso that
the aerosol composition contains, based on the weight thereof, 0.1 to 10 wt.% copolymer(s) of N-octylacrylamide/acrylic acid/tert-butylaminoethyl methacrylate (INCl names: octylacrylamide/acrylates/butylaminoethyl methacrylate copolymer).

2. The hairspray according to claim 1, **characterized in that** the aerosol composition contains, based on the weight thereof, 20 to 80 wt.%, preferably 22.5 to 77.5 wt.%, more preferably 25 to 75 wt.%, still more preferably 27.5 to 72.5 wt.% and in particular 30 to 70 wt.%, a propellant or a propellant mixture selected from dimethyl ether, HFO-1234yf, HFO-1234ze, propane, propene, n-butane, isobutane, isobutene, n-pentane, pentene, isopentane and isopentane or mixtures thereof.

3. The hairspray according to either claim 1 or claim 2, **characterized in that** it contains, as the sole propellant, propane, propene, n-butane, isobutane, isobutene, n-pentane, pentene, isopentane, isopentene, dimethyl ether or mixtures of the above-mentioned hydrocarbons with dimethyl ether.

4. The hairspray according to one of claims 1 to 3, **characterized in that** the aerosol composition contains, based on the weight thereof, setting polymer(s) in a total amount of from 0.2 wt.% to 17.5 wt.%, preferably from 0.5 wt.% to 15 wt.%, particularly preferably from 2.0 wt.% to 10.0 wt.% and in particular from 3.0 to 5.0 wt.%, based on the weight of the agent in each case.

5. The hairspray according to one of claims 1 to 4, **characterized in that** the aerosol composition contains at least one setting polymer selected from
- non-ionic polymers based on ethylenically unsaturated monomers, in particular from
- homopolymers of N-vinylpyrrolidone,
- non-ionic copolymers of N-vinylpyrrolidone,
- homopolymers and non-ionic copolymers of N-vinylcaprolactam,
- copolymers of (meth)acrylamide,
- polyvinyl alcohol, polyvinyl acetate,
- chitosan and chitosan derivatives,
- cationic cellulose derivatives,
- cationic copolymers of 3-(C₁ to C₆)-alkyl-1-vinyl-imidazolinium,
- homopolymers and copolymers containing the structural unit of formula (M-1) 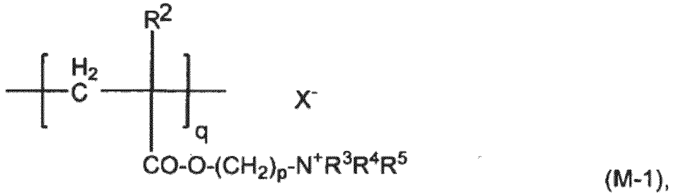 in which: R² represents -H or -CH₃; R³, R⁴ and R⁵ are selected, independently of one another, from (C₁ to C₄)-alkyl groups, (C₁ to C₄)-alkenyl groups or (C₂ to C₄)-hydroxyalkyl groups; p represents 1, 2, 3 or 4; q represents a natural number; and X- represents a physiologically acceptable organic or inorganic anion,
- anionic polymers comprising carboxylate and/or sulfonate groups, and
- anionic polyurethanes.

6. The hairspray according to one of claims 1 to 5, **characterized in that** it contains at least one film-forming and/or setting polymer selected from
- aminomethyl propanol salts of copolymers of allyl methacrylate having one or more monomers selected from acrylic acid, methacrylic acid, acrylic acid esters and methacrylic acid esters,
- vinylpyrrolidone-vinyl acetate copolymers,
- vinylpyrrolidone-vinylcaprolactam-dimethylaminopropyl acrylamide copolymers,
- copolymers of octylacrylamide having t-butylaminoethyl methacrylate and two or more monomers selected from acrylic acid, methacrylic acid, acrylic acid esters and methacrylic acid esters, and
- copolymers of C₁₋₂-alkyl succinates having hydroxyalkyl acrylates and one or more monomers selected from acrylic acid, methacrylic acid, acrylic acid esters and methacrylic acid esters.

7. The hairspray according to one of claims 1 to 6, **characterized in that** it contains as a film-forming or setting polymer selected from an aminomethyl propanol salt of a copolymer of allyl methacrylate having one or more monomers selected from acrylic acid, methacrylic acid, acrylic acid esters and methacrylic acid esters.

8. The hairspray according to one of claims 1 to 7, **characterized in that** the aerosol composition contains, based on the weight thereof, 0.25 to 9 wt.%, more preferably 0.5 to 8 wt.%, particularly preferably 0.75 to 7 wt.% and in particular 1 to 6 wt.%, copolymer(s) from N-octylacrylamide/acrylic acid/tert-buylaminoethyl methacrylate (INCl names: octylacrylamide/acrylates/butylaminoethyl methacrylate copolymer).

9. The hairspray according to one of claims 1 to 8, **characterized in that** the aerosol composition contains, based on the weight thereof, 0.11 to 4.5 wt.%, preferably 0.12 to 4 wt.%, more preferably 0.13 to 3 wt.%, particularly preferably 0.14 to 2.5 wt.% and in particular 0.15 to 2 wt.%, esters of formula (I).

10. The hairspray according to one of claims 1 to 9, **characterized in that** the aerosol composition contains, based on the weight thereof, 0.11 to 4.5 wt.%, preferably 0.12 to 4 wt.%, more preferably 0.13 to 3 wt.%, particularly preferably 0.14 to 2.5 wt.% and in particular 0.15 to 2 wt.%, esters of formula (Ia) 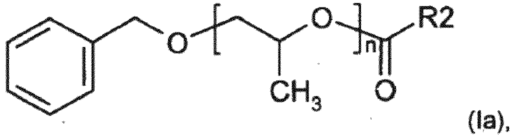 in which
R2 represents a straight-chain or branched alkyl functional group having 7 to 15 carbon atoms,
n represents an integer from the group of 1, 2, 3, 4, 5, 6, 7 and 8.

11. The hairspray according to one of claims 1 to 10, **characterized in that** the aerosol composition contains, based on the weight thereof, 0.11 to 4.5 wt.%, preferably 0.12 to 4 wt.%, more preferably 0.13 to 3 wt.%, particularly preferably 0.14 to 2.5 wt.% and in particular 0.15 to 2 wt.%, esters of formula (Ib) 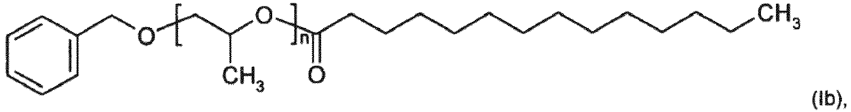 in which n represents an integer from the group of 1, 2, 3 or 4, preferably represents 2 or 3, and in particular represents 3.

12. The hairspray according to one of claims 1 to 11, **characterized in that** the aerosol composition contains, based on the weight thereof, 0.11 to 4.5 wt.%, preferably 0.12 to 4 wt.%, more preferably 0.13 to 3 wt.%, particularly preferably 0.14 to 2.5 wt.% and in particular 0.15 to 2 wt.%, esters of formula (Ic) 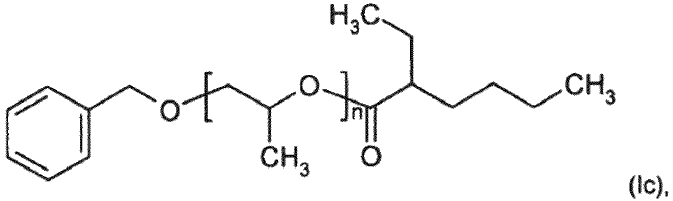 in which n represents an integer from the group of 1, 2, 3 or 4, preferably represents 2 or 3, and in particular represents 3.

13. A method for styling hair, **characterized in that** an aerosol composition according to claim 1 containing esters of formula (I) 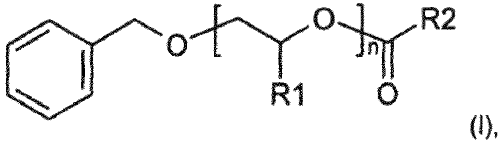 in which
R1 represents -H or -CH₃,
R2 represents a straight-chain or branched alkyl functional group having 7 to 15 carbon atoms, and
n represents an integer from the group of 1, 2, 3, 4, 5, 6, 7 and 8,
is applied to keratin fibers from a pressurized container by means of a propellant or a propellant mixture.

## Revendications

1. Vaporiseur capillaire comprenant un récipient sous pression et, à l'intérieur, une composition aérosol qui contient, sur la base de son poids,
a. 20 à 80 % en poids d'au moins un agent propulseur ;
b. 0,1 à 20 % en poids d'au moins un polymère raffermissant ;
c. 0,01 à 5 % en poids d'esters de la formule (I) 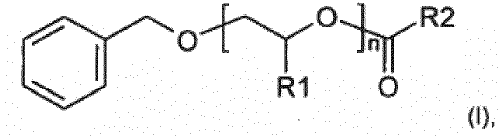
dans laquelle
R1 est -H ou -CH₃,
R2 représente un radical alkyle linéaire ou ramifié comportant 7 à 15 atomes de carbone,
n est un entier du groupe comprenant 1, 2, 3, 4, 5, 6, 7, 8,
à condition que
la composition aérosol contienne, sur la base de son poids, de 0,1 à 10 % en poids d'au moins un copolymère parmi N-octylacrylamide/acide acrylique/méthacrylate de tert-butylaminoéthyle (nom INCl: Octylacrylamide/Acrylates/Butylamino Methacrylate Copolymer).

2. Vaporiseur capillaire selon la revendication 1, **caractérisé en ce que** la composition aérosol contient, sur la base de son poids, de 20 à 80 % en poids, de préférence de 22,5 à 77,5 % en poids, plus préférablement de 25 à 75 % en poids, encore plus préférablement de 27,5 à 72,5 % en poids et en particulier de 30 à 70 % en poids d'agent propulseur ou de mélange d'agents propulseurs, choisis parmi l'éther de diméthyle, le HFO1234yf, le HFO1234ze, le propane, le propène, le n-butane, l'iso-butane, l'iso-butène, le n-pentane, le pentène, l'isopentane et l'iso-pentène ou des mélanges de ceux-ci.

3. Vaporisateur capillaire selon l'une des revendications 1 ou 2, **caractérisé en ce qu'**il contient du propane, du propène, du n-butane, de l'iso-butane, de l'iso-butène, du n-pentane, du pentène, de l'iso-pentane et de l'iso-pentène, de l'éther de diméthyle ou des mélanges desdits hydrocarbures avec du diméthyléther comme seul agent propulseur.

4. Vaporisateur capillaire selon l'une des revendications 1 à 3, **caractérisé en ce que** la composition aérosol contient, sur la base de son poids, au moins un polymère raffermissant dans une quantité totale allant de 0,2 % en poids à 17,5 % en poids, de préférence de 0,5 % en poids à 15 % en poids, plus préférablement de 2,0 % en poids à 10,0 % en poids et de manière particulièrement préférée de 3,0 % à 5,0 % en poids, à chaque fois rapportée au poids de l'agent.

5. Vaporisateur capillaire selon l'une des revendications 1 à 4, **caractérisé en ce que** la composition aérosol contient au moins un polymère raffermissant choisi parmi
- les polymères non ioniques à base de monomères à insaturation éthylénique, notamment
- d'homopolymères de la N-vinylpyrrolidone,
- de copolymères non ioniques de la N-vinylpyrrolidone,
- d'homopolymères et copolymères non ioniques du N-vinylcaprolactame,
- de copolymères de (méth)acrylamide,
- d'alcool polyvinylique, d'acétate de polyvinyle,
- de chitosane et de dérivés du chitosane,
- de dérivés de cellulose cationiques,
- de copolymères cationiques du 3-(alkyle en C₁ à C₆)-1-vinyl-imidazolinium,
- d'homopolymères et de copolymères contenant l'unité structurale de la formule (M-1) 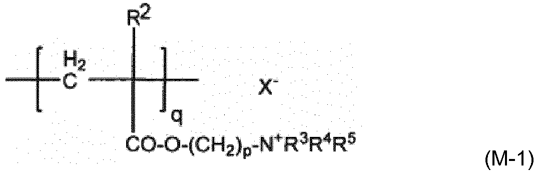 dans laquelle R² = -H ou -CH₃, R³, R⁴ et R⁵ sont choisis indépendamment parmi les groupes d'alkyle en C₁ à C₄, alcényle en C₁ à C₄ ou hydroxyalkyle en C₂ à C₄, p = 1, 2, 3 ou 4, q est un nombre naturel et X⁻ est un anion organique ou minéral physiologiquement acceptable,
- de polymères anioniques comportant des groupes carboxylate et/ou sulfonate,
- de polyuréthanes anioniques.

6. Vaporisateur capillaire selon l'une des revendications 1 à 5, **caractérisé en ce qu'**il contient au moins un polymère filmogène et/ou raffermissant choisi parmi
- les sels d'aminométhylpropanol de copolymères de méthacrylate d'allyle avec au moins un monomère choisi parmi l'acide acrylique, l'acide méthacrylique, les acrylates et les méthacrylates,
- les copolymères vinylpyrrolidone-acétate de vinyle,
- les copolymères vinylpyrrolidone-caprolactame de vinyle-acrylamide de diméthylaminopropyle,
- les copolymères de l'acrylamide d'octyle avec le méthacrylate de t-butylaminoéthyle et d'au moins deux monomères choisis parmi l'acide acrylique, l'acide méthacrylique, les acrylates et les méthacrylates, et
- les copolymères de succinates d'alkyle en C₁ à C₂ avec des acrylates d'hydroxyalkyle et d'au moins un monomère choisi parmi l'acide acrylique, l'acide méthacrylique, les acrylates et les méthacrylates.

7. Vaporisateur capillaire selon l'une des revendications 1 à 6, **caractérisé en ce qu'**il contient comme polymère filmogène et/ou raffermissant un sel d'aminométhylpropanol d'un copolymère du méthacrylate d'allyle avec au moins un monomère choisi parmi l'acide acrylique, l'acide méthacrylique, les acrylates et les méthacrylates.

8. Vaporisateur capillaire selon l'une des revendications 1 à 7, **caractérisé en ce que** la composition aérosol contient, sur la base de son poids, de 0,25 à 9 % en poids, plus préférablement de 0,5 à 8 % en poids, de manière particulièrement préférée de 0,75 à 7 % en poids et en particulier de 1 à 6 % d'au moins un copolymère N-octylacrylamide/acide acrylique/méthacrylate de tert-butylaminoéthyle (nom INCl : Octylacrylamide/Acrylates/Butylaminoethyl Methacrylate Copolymère).

9. Vaporisateur capillaire selon l'une des revendications 1 à 8, **caractérisé en ce que** la composition aérosol contient, sur la base de son poids, de 0,11 à 4,5 % en poids, de préférence de 0,12 à 4 % en poids, plus préférablement de 0,13 à 3 % en poids, de manière particulièrement préférée 0,14 à 2,5 % en poids et en particulier de 0,15 à 2 % en poids d'ester de la formule (I).

10. Vaporisateur capillaire selon l'une des revendications 1 à 9, **caractérisé en ce que** la composition aérosol contient, sur la base de son poids, de 0,11 à 4,5 % en poids, de préférence de 0,12 à 4 % en poids, plus préférablement de 0,13 à 3 % en poids, de manière particulièrement préférée de 0,14 à 2,5 % en poids et en particulier de 0,15 à 2 % en poids d'ester de la formule (la) 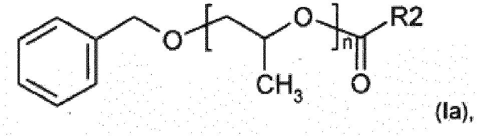 dans laquelle
R2 représente un radical alkyle linéaire ou ramifié comportant 7 à 15 atomes de carbone,
n est un entier du groupe 1, 2, 3, 4, 5, 6, 7, 8.

11. Vaporisateur capillaire selon l'une des revendications 1 à 10, **caractérisé en ce que** la composition aérosol contient, sur la base de son poids, de 0,11 à 4,5 % en poids, de préférence de 0,12 à 4 % en poids, plus préférablement de 0,13 à 3 % en poids, de manière particulièrement préférée de 0,14 à 2,5 % en poids et en particulier de 0,15 à 2 % en poids d'esters de la formule (Ib) 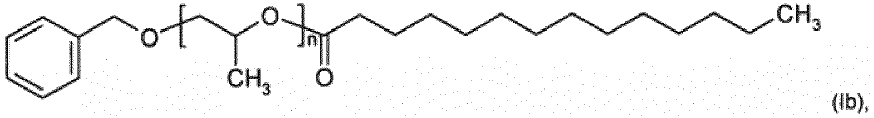 dans laquelle n un nombre entier du groupe 1, 2, 3 ou 4, de préférence 2 ou 3 et en particulier 3.

12. Vaporisateur capillaire selon l'une des revendications 1 à 11, **caractérisé en ce que** la composition aérosol contient, sur la base de son poids, de 0,11 à 4,5 % en poids, de préférence de 0,12 à 4 % en poids, plus préférablement de 0,13 à 3 % en poids, de manière particulièrement préférée de 0,14 à 2,5 % en poids et en particulier de 0,15 à 2 % en poids d'ester de la formule (Ic) 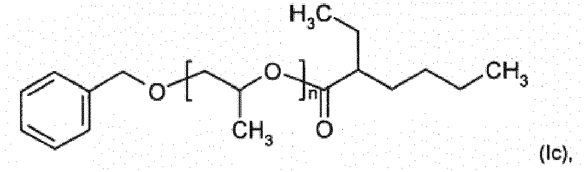 dans laquelle n est un entier du groupe comportant 1, 2, 3 ou 4, de préférence 2 ou 3 et en particulier 3.

13. Procédé de coiffage **caractérisé en ce qu'**une composition aérosol selon la revendication 1, laquelle contient l'ester de la formule (I) 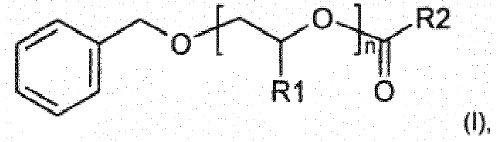 dans laquelle
R1 représente -H ou -CH₃,
R2 représente un radical alkyle linéaire ou ramifié comportant 7 à 15 atomes de carbone,
n est un entier du groupe 1, 2, 3, 4, 5, 6, 7, 8
est appliquée sur des fibres de kératine depuis un récipient sous pression au moyen d'un agent propulseur ou d'un mélange d'agents propulseurs.
